(19) Europäisches Patentamt
European Patent Office
Office européen des brevets

(11) EP 0 841 331 B1

(12) FASCICULE DE BREVET EUROPEEN

(45) Date de publication et mention de la délivrance du brevet: **08.01.2003 Bulletin 2003/02**

(51) Int Cl.[7]: **C07D 303/40**, C07C 69/608, C07C 67/313

(21) Numéro de dépôt: **97118603.6**

(22) Date de dépôt: **25.10.1997**

(54) **Epoxydes nouveaux, procédé pour leur préparation et leur utilisation pour la préparation d'ingrédients parfumants**

Neue Epoxiden, Verfahren zu ihrer Herstellung und ihre Verwendung für die Herstellung von Riechstoff-Bestandteilen

New epoxides, process for their preparation and their use for the preparation of perfume ingredients

(84) Etats contractants désignés:
**CH DE ES FR GB IT LI NL**

(30) Priorité: **07.11.1996 CH 275096**

(43) Date de publication de la demande:
**13.05.1998 Bulletin 1998/20**

(73) Titulaire: **FIRMENICH SA**
**1211 Genève 8 (CH)**

(72) Inventeur: **Fehr, Charles**
**1290 Versoix (CH)**

(74) Mandataire: **Salvaterra-Garcia, Maria de Lurdes**
**Firmenich SA**
**Département des Brevets**
**Case Postale 239**
**1211 Genève 8 (CH)**

(56) Documents cités:
**WO-A-95/33735**

- **T. KITAHARA ET. AL.: "Synthesis of Methyl Jasmonate and Methyl Cucurbate." AGRICULTURAL AND BIOLOGICAL CHEMISTRY, vol. 51, no. 4, avril 1987 (1987-04), pages 1129-33, XP002053538**
- **T. KITAHARA ET. AL.: "A Simple and Efficient Synthesis of Methyl Dihydroepijasmonate." AGRICULTURAL AND BIOLOGICAL CHEMISTRY, vol. 50, no. 7, juillet 1986 (1986-07), pages 1867-72, XP002053539**
- **E. J. COREY ET. AL.: "Enantioselective Route to a Key Intermediate in the Total Synthesis of Ginkgolide B." TETRAHEDRON LETTERS, vol. 29, no. 26, 1988, pages 3201-4, XP002053540**
- **D. P CURRAN ET. AL.: "Radical-Initiated Polyolefinic Cyclisations in Linear Triquinane Synthesis. Model Studies and Total Synthesis of Hirsutene." TETRAHEDRON, vol. 41, no. 19, 1985, pages 3943-58, XP002053541**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 841 331 B1

Printed by Jouve, 75001 PARIS (FR)

## Description

La présente invention a trait au domaine de la synthèse organique. Elle concerne plus particulièrement des époxydes utiles en tant que produits de départ pour la préparation d'ingrédients parfumants très prisés, parmi lesquels les isomères de l'Hédione® (3-oxo-2-pentyl-1-cyclopentaneacétate de méthyle ; origine : Firmenich SA, Genève, Suisse) qui sont préférés d'un point de vue olfactif.

Ainsi, un objet de la présente invention est un époxyde de formule

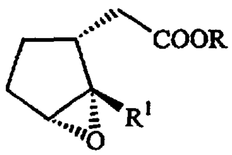

(I)

ayant une configuration cyclanique (1R), le groupe en position 2 étant en configuration trans et le groupe époxy étant en configuration cis par rapport à celui en position 1 du cycle, et dans laquelle R représente un radical alkyle inférieur et $R^1$ représente un radical d'hydrocarbure, saturé ou insaturé, linéaire ou ramifié, ayant de 1 à 8 atomes de carbone.

Par radical alkyle inférieur on entend ici un radical alkyle linéaire ou ramifié, de $C_1$ à $C_4$, et plus particulièrement le radical méthyle ou éthyle.

Les époxydes de formule (I) sont des composés de structure nouvelle.

La demande de brevet international WO 95/33735, publiée le 14 décembre 1995, décrit des époxydes de formule

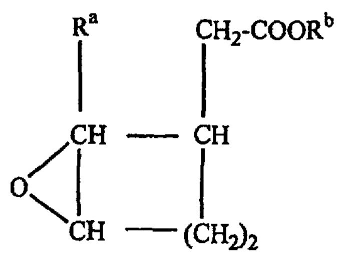

(A)

dans laquelle $R^a$ représente un groupe alkyle ayant de 1 à 8 atomes de carbone et $R^b$ un groupe alkyle de $C_1$ à $C_4$. On ne peut cependant y déceler une description ou mention quelconque de la stéréochimie précise des composés (A). Or, en essayant de reproduire le procédé décrit dans ce document pour la préparation de ces composés, nous avons découvert qu'il ne permettait d'obtenir qu'un mélange de diastéréomères de l'époxyde de formule (A) dans laquelle $R^a$ est un radical pentyle et $R^b$ un radical méthyle. Nous avons donc constaté que le procédé de préparation des époxydes (A) décrit dans WO 95/33735 n'était pas diastéréosélectif, la réaction se déroulant par ailleurs avec un taux de conversion très bas, de l'ordre de 25%.

Nous avons également découvert que, contrairement à ce qui est indiqué dans WO 95/33735, la transformation de l'époxyde ainsi obtenu, dans les conditions de réaction décrites, à savoir le traitement thermique de l'époxyde en présence d'une quantité catalytique d'iodure de lithium, menait en fait à la formation d'un produit entièrement distinct de la cétone voulue. Ainsi, lorsque nous avons appliqué le procédé décrit dans WO 95/33735 à l'époxyde de formule (A) ayant $R^a$ = pentyle et $R^b$ = $CH_3$, nous avons obtenu, au lieu du 3-oxo-2-pentyl-1-cyclopentaneacétate de méthyle attendu selon la description de ce document, une lactone entièrement distincte identifiée de par ses données spectroscopiques, comme ayant la structure

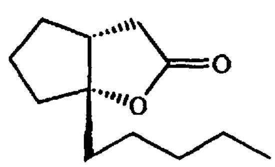

à savoir la cis-perhydro-6a-pentyl-2-cyclopenta[b]furanone.

Il s'agit en fait d'une lactone connue de longue date dans nos laboratoires, dotée d'une odeur aqueuse-métallique, chimique, aussi légèrement animale. Par contre, aucune trace de la cétone cyclique désirée n'a pu être décelée dans ce produit. Le même résultat a été constaté dans les cas où $R^a$ = hexyle et $R^b$ = méthyle ou éthyle.

En somme, il ressort de ce qui précède que le document de l'art antérieur susmentionné ne décrit pas un procédé permettant d'obtenir ni de transformer les époxydes de formule (I) selon la présente invention et, de ce fait même, n'a pas reconnu un intérêt quelconque dans les composés ayant la stéréochimie particulière indiquée et faisant l'objet de la présente invention. Or, nous venons de découvrir que les composés (I) se révèlent d'une grande utilité en tant que produits de départ pour la préparation, à l'état pur, d'ingrédients parfumants optiquement actifs particulièrement prisés par les parfumeurs.

Il convient également de noter que, comme il ressort des exemples de comparaison présentés plus loin, le document de l'art antérieur susmentionné ne décrit pas non plus les racémates purs correspondant aux époxydes (I), c'est-à-dire les époxydes de formule

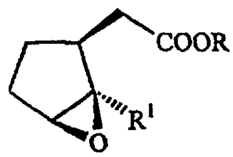

(I')

dans laquelle les groupes substituants époxy et carboxy ont une configuration relative strictement cis et les symboles R et $R^1$ ont le sens indiqué à la formule (I). Ces composés, qui sont des mélanges équimolaires d'un époxyde (I) et de son énantiomère respectif, sont ainsi également des composés de structure nouvelle. Ils se révèlent utiles pour la préparation des cétones correspondantes ayant une configuration cyclanique strictement cis, dont l'exemple le plus intéressant pour la parfumerie est la cis-Hédione® ou cis-3-oxo-2-pentyl-1-cyclopentaneacétate de méthyle.

Selon l'invention, les époxydes optiquement actifs de formule (I) sont des composés préférés et on peut citer, à titre encore plus préférentiel, le (+)-(1R,2S,3R)-2,3-époxy-2-pentyl-1-cyclopentaneacétate de méthyle. La transformation de ce composé selon le procédé original décrit ci-après permet de préparer la (+)-cis-Hédione®, ou (+)-(1R)-cis-3-oxo-2-pentyl-1-cyclopentaneacétate de méthyle, c'est-à-dire l'isomère que l'on sait posséder au mieux les caractères odorants, notamment la note jasminée, typiques de cette cétone cyclique réputée.

Ainsi, l'invention se rapporte également à l'utilisation des époxydes de formule (I) pour la préparation des cétones cycliques correspondantes de formule

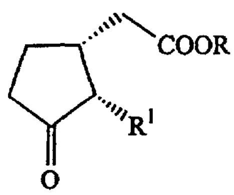

(II)

ayant une configuration cyclanique (1R)-cis, selon un procédé de transformation original, caractérisé par le traitement dudit époxyde dans un solvant organique inerte, avec un agent acide constitué par un acide de Lewis approprié ou une terre argileuse acide.

Nous avons découvert avec surprise que ces conditions de réaction permettaient d'obtenir les cétones optiquement actives de formule (II) de façon sélective et avec des rendements excellents, comme il ressort des exemples présentés plus loin. Ce résultat est contraire à celui que l'on a pu observer avec le procédé de l'art antérieur décrit dans le document cité plus haut, selon lequel l'époxyde était soumis à un traitement thermique en présence d'un sel de métal alcalin ou alcalino-terreux d'un acide de bas poids moléculaire.

La réaction qui caractérise le procédé de l'invention a lieu à des températures très variées qui dépendent des réactifs utilisés et notamment de la réactivité de l'agent acide. Ainsi, et contrairement au procédé décrit dans WO 95/33735, le procédé selon la présente invention permet l'utilisation de conditions de températures douces, typiquement inférieures à 120°C.

En tant qu'acide de Lewis approprié pour le procédé de l'invention, on peut citer notamment le trifluorure de bore, éventuellement complexé (par exemple comme éthérate), le trichlorure d'aluminium et l'iodure de magnésium.

En tant que terres argileuses acides, on peut utiliser les argiles à base d'aluminosilicates appartenant à la

famille des produits connus sous le nom de FILTROL® (origine: Harshaw/Filtrol), celles disponibles commercialement sous des désignations telles que GK (origine : Georgia Kaolin Co.), les montmorillonites connues sous le nom de catalyseurs K, par exemple K10, KP10, KSF et KSF/O (origine: Süd-Chemie AG), les bentonites, ou encore toute autre terre argileuse acide d'usage courant. Selon un mode d'exécution préférentiel de l'invention, on utilisera par exemple le Filtrol® G24.

La proportion dans laquelle l'agent acide peut être utilisé, par rapport à l'époxyde de départ, varie dans une gamme de concentrations très large. Nous avons observé que le rapport entre les deux réactifs pouvait être par exemple stoïchiométrique, mais que des résultats tout aussi utiles étaient obtenus avec des quantités catalytiques d'agent acide, par rapport à l'époxyde.

En tant que solvant pouvant être utilisé selon l'invention, on peut employer un solvant d'usage courant qui soit inerte dans les conditions de la réaction. On peut citer notamment les hydrocarbures cycliques ou acycliques, en particulier le cylohexane, le xylène et le toluène, les éthers tels que le diéthyl éther ou le tétrahydrofurane, ou encore les solvants chlorés, notamment le dichlorométhane. Des résultats particulièrement utiles ont été obtenus avec le toluène ou le dichlorométhane.

Selon un mode d'exécution préféré de ce procédé, on utilise en tant que produit de départ le (+)-(1R,2S,3R)-2,3-époxy-2-pentyl-1-cyclopentaneacétate de méthyle. Ce mode d'exécution se révèle particulièrement avantageux car, comme cité plus haut, il permet d'obtenir l'isomère préféré de l'Hédione®, à savoir le (+)-(1R)-cis-3-oxo-2-pentyl-1-cyclopentaneacétate de méthyle.

Bien entendu, si on utilise selon le procédé décrit ci-dessus un époxyde racémique de formule (I') définie plus haut, on obtiendra les cétones cycliques racémiques correspondantes, qui sont les équivalents racémiques, de configuration cyclanique strictement cis, des cétones de formule (III). Ainsi, en utilisant, par exemple, le c-2,3-époxy-2-pentyl-r-1-cyclopentaneacétate de méthyle, on obtiendra selon l'invention la cis-Hédione® racémique à l'état pur.

L'invention concerne également un procédé pour la préparation des époxydes de formule (I) définie plus haut, caractérisé en ce qu'on fait réagir un ester de formule

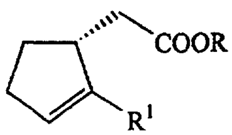

(III)

ayant une configuration cyclanique (1R) et dans laquelle les symboles R et $R^1$ ont le sens indiqué à la formule (I), avec un peracide fort, dans un solvant organique inerte.

Les esters optiquement actifs de formule (III) sont des composés nouveaux, préparés selon un procédé original décrit plus loin.

En effet, bien que l'on connaisse de l'article de T. Kitahara et al., dans Agric. Biol. Chem. 50, 1867 (1986), le 2-pentyl-2-cyclopentène-1-acétate de méthyle racémique, il n'est nullement fait mention dans cet article de l'un ou l'autre des énantiomères présents dans ce composé racémique, notamment celui de configuration (1R) qui obéit à la formule (III). Or, comme on le verra plus loin, la présente invention fournit un procédé original pour la préparation des énantiomères désirés de formule (III) ayant la configuration absolue (1R), et plus particulièrement du (+)-(1R)-2-pentyl-2-cyclopentène-1-acétate de méthyle.

En tant que peracide fort utilisé selon l'invention dans la préparation des époxydes de formule (I), on peut employer des peracides organiques fortement électro-attracteurs, choisis parmi les peroxyacides d'usage courant dans des réactions d'oxidation (voir, par exemple, chapitre III dans Org. Chem., 5, ed. W.S. Trahanovsky, Academic Press, 1978). On citera dans ce contexte, à titre préférentiel, les acides permaléique, perphtalique, m-chloroperbenzoïque ou encore les acides de formule $CX_3CO_2OH$, X représentant typiquement un atome d'halogène, notamment le chlore ou le fluor. De préférence, on utilisera l'acide permaléique ou l'acide trifluoroperacétique.

En tant que solvant approprié dans ce procédé, on utilisera de préférence un solvant chloré. Des résultats très utiles ont été obtenus avec le dichlorométhane en particulier.

Les esters de formule (III) utilisés en tant que produits de départ dans le procédé décrit ci-dessus sont des composés nouveaux, qui font également l'objet de l'invention. Parmi ces esters, on peut citer notamment le (+)-(1R)-2-pentyl-2-cyclopentène-1-acétate de méthyle, dont la transformation selon l'invention permet d'obtenir l'isomère préféré de l'Hédione®.

L'invention se rapporte également à un procédé original pour préparer les esters de formule (III), à partir de produits disponibles commercialement ou pouvant être préparés au moyen de réactions courantes. Les réactions qui caractérisent le procédé de préparation des esters (III) sont représentées schématiquement ci-après

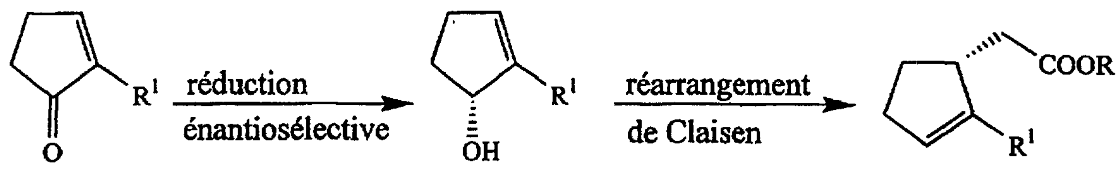


(V) (IV) (III)

Selon l'invention, la première réaction dans ce schéma consiste en une réduction énantiosélective de la cyclopenténone de départ, effectuée à l'aide d'un système de type oxazaborolidine-borane, qui peut être utilisé en quantités stoïchiométriques ou catalytiques, par rapport au substrat Il s'agit d'un système de réduction énantiosélective de cétones décrit dans plusieurs publications (voir, par exemple, E.J. Corey et al., J. Amer. Chem. Soc. 1987, 109, 7925; S. Itsuno et al., Bull. Chem. Soc. Jpn, 1987, 60, 395 ; D.J. Mathre et al., J. Org. Chem. 1991, 56, 751; V.K. Singh, Synthesis 1992, 605), mais qui, à notre connaissance, n'a jamais été utilisé dans la réduction de cyclopenténones telles que citées plus haut. Il implique l'emploi de l'oxazaborolidine de formule

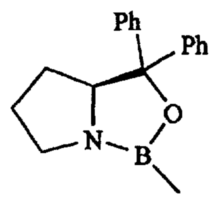

Ph = phényle

ou (S)-tétrahydro-1-méthyl-3,3-diphényl-1H,3H-pyrrolo[1,2-c][1,3,2]oxazaborole en tant que catalyseur de réduction, la réduction étant effectuée à l'aide de $BH_3$ (sous forme complexée). La réaction de réduction produit, en plus de l'alcool (IV) désiré, le (S)-$\alpha$,$\alpha$-diphényl-2-pyrrolidineméthanol, lequel peut être récupéré et reconverti en l'oxazaborolidine susmentionnée de façon connue en soi (voir D.J. Mathre, ref. citée).

Selon un mode d'exécution plus avantageux, l'oxazaborolidine susmentionnée est utilisée en quantités catalytiques par rapport à la cyclopenténone (V), pour fournir les alcools (IV) avec une pureté optique d'au moins 90% e.e. Les conditions détaillées de ces réactions sont décrites plus loin.

Nous avons aussi réussi à préparer un nouveau système de catalyseur pour la réduction mentionnée ci-dessus selon la formule suivante

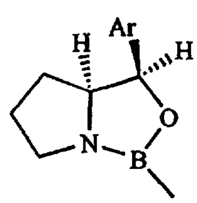

(VI) Ar = aryle

dans laquelle, par rapport aux catalyseurs de type oxazaborolidines connus et décrits auparavant, un des groupes phényles est remplacé par un atome d'hydrogène. Dans la formule ci-dessus, le substituant aryle peut être un groupe phényle, substitué ou non-substitué, comme par exemple le toluène, le xylène, le di-tert-butylbenzène ou le mésitylène, ou même un groupe aryle condensé, comme le naphtalène.

Le nouveau système de catalyseur peut être synthétisé par une réaction analogue à celle utilisée pour la préparation des catalyseurs oxazaborolidines connus portant deux groupes phényles, cette réaction utilisant, en tant que produit de départ, l'amino-alcool de formule

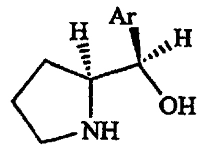

dans laquelle Ar a le sens indiqué ci-dessus. Les amino-alcools sont préparés selon un procédé connu (voir A. Ookawa, K. Soai, J. Chem. Soc. Perkin Trans. 1, 1987, 1465) et ensuite on les fait réagir avec du triméthylboroxine $(BOMe)_3$ pour donner le catalyseur souhaité.

Lesdits catalyseurs sont de nouvelles entités chimiques qui sont également un objet de l'invention.

En utilisant le nouveau système de catalyseur dans lequel Ar = phényle, à savoir le (S)-tétrahydro-1-méthyl-3-phényl-1H,3H-pyrrolo[1,2-c][1,3,2]oxazaborole dans la réduction énantiosélective de la présente invention, nous avons obtenu des résultats qui étaient aussi bons que ceux obtenus avec les oxazaborolidines connus cités auparavant. Comme ces derniers, les nouveaux catalyseurs peuvent être utilisés en quantités stoéchiométriques ou catalytiques par rapport aux cyclopenténones (V).

Une autre voie synthétique pour une réduction énantiosélective des cyclopenténones de la formule (V) en alcools (IV) comprend une étape biotechnologique. Lesdites cétones peuvent, d'une façon conventionnelle, être converties en les acétates racémiques correspondants, et les derniers être ensuite saponifiés énantiosélectivement en alcools désirés et optiquement actifs (IV) en utilisant une lipase.

Des lipases qui sont appropriées pour une utilisation dans cette étape de réaction sont connues de l'homme de l'art et comprennent, par exemple *Candida antarctica, Pseudomonas fluorescens, Pseudomonas Amano, Humicola lang., Candida cylindracea, Mucor Miehei, Chromabacterium viscosum, Aspergillus niger, Mucor javanicus and Rhisopus arrhizus.*

La transformation de la fonction cétonique en fonction acétate peut être effectuée en utilisant des agents dont l'utilisation est courante dans le métier. Dans ce contexte, on peut citer le système $LiAlH_4$/anhydride acétique, mais d'autres systèmes peuvent être utilisés. De même, des esters autres que l'acétate peuvent être utilisés dans le procédé.

Le réarrangement de Claisen des alcools allyliques (III) a permis d'obtenir les esters de formule (III) avec rétention totale de la configuration énantiomérique. Il s'agit là d'un résultat bien surprenant, étant donné que l'on aurait pu s'attendre à un degré de racémisation considérable du cyclopenténol de départ via déshydratation réversible favorisée par les conditions acides et de température élevée du milieu de réaction. Or, nous n'avons observé pratiquement aucune perte (moins de 1% de racémisation) dans la pureté optique du produit obtenu, par rapport à celle du penténol de départ

Les conditions de ces réactions d'estérification sont décrites en détail plus loin.

En somme, la présente invention permet la préparation d'époxydes optiquement actifs nouveaux, qui sont eux-mêmes utiles pour la préparation d'ingrédients parfumants précieux, et qui permettent d'obtenir ces derniers avec des rendements très utiles et une pureté diastéréochimique et énantiomérique excellente. Ceci, tout en faisant appel à des réactions qui peuvent être appliquées à l'échelle industrielle, sans risque et sans danger pour l'environnement.

L'invention sera maintenant décrite de façon plus détaillée, à l'aide des exemples suivants, dans lesquels les températures sont indiquées en degrés Celsius et les abréviations ont le sens usuel dans l'art.

Exemple 1

Préparation du (+)-(1R)-2-pentyl-1-cyclopentène-1-cyclopentène-1-ol

Une solution de la 2-pentyl-2-cyclopentén-1-one (9,12 g, 60 mmole) dans 140 ml de tétrahydrofurane (THF) a été traitée avec l'oxazaborolidine représentée plus haut, à savoir le (S)-tétrahydro-1-méthyl-3,3-diphényl-1H,3H-pyrrolo[1,2-c][13,2]oxazaborole (16,7 ml ; 0,36 M dans le toluène, 6,0 mmole ; voir D.J. Mathre, ref. citée). On a ajouté, à 0° et goutte-à-goutte, une solution de $BH_3.S(CH_3)_2$ (3,30 ml = 2,64 g, 34,8 mmole) dans le THF (60 ml), pendant 1 h. La réaction a été arrêtée avec 3 ml de méthanol (libération de $H_2$), suivi d'éther éthylique et HCl à 5%. On a séparé la phase organique, qui a été lavée jusqu'à neutralité (2 fois à l'eau et une fois avec de la saumure) et évaporée. On a obtenu 9,5 g de produit brut qui a été distillé au four à boules (temp. four 60°/11 Pa) pour fournir 8,1 g de (+)-(1R)-2-pentyl-2-cyclopentén-1-ol (rend. 89%).

Ce composé présentait les données analytiques suivantes.

$$[\alpha]^{20}_{D} = +28,3 \ (c = 2,7 \ ; CHCl_3)$$

excès énantiomérique (e.e.) = 91% (mesuré sur colonne chirale de type Megadex® 5 ou CD-Chirasil® DEX CB sur la base de l'éther triméthylsilylique correspondant)
RMN($^1$H, 360MHz) : 0,89(t, J=7, 3H) ; 1,20-1,40(m, 4H) ; 1,40-1,57(m, 3H ; avec $D_2O$ = 2H) ; 1,69(m, 1H) ; 2,02-2,47(m, 5H) ; 4,64(large, 1H) ; 5,53(large s, 1H) δppm.
RMN($^{13}$C) 146,7(s); 126,9(s) ; 78,9(d) ; 34,1(t) ; 31,9(t); 29,7(t); 28,1(t); 27,5(t); 22,6(t); 14,1(q) δppm.
SM : 154($M^+$, 2), 97(30), 83(100), 79(12), 67(7), 55(11), 41(9).

En utilisant une autre oxazaborolidine, à savoir le (S)-tétrahydro-1-méthyl-3-phényl-1H,3H-pyrrolo-[1,2-c][1,3,2]oxazaborole (voir Exemple 7) en tant que catalyseur dans la réduction asymmétrique, et dans une proportion de 10% par rapport au substrat, le même cyclopenténol chiral a été obtenu avec un rendement de 68% et un e.e. de 90%.

Exemple 2

Préparation du (+)-(1R)-2-pentyl-1-cyclopentène-1-acétate d'éthyle

Un mélange du cyclopenténol obtenu selon l'Exemple 1 (5 g, 32,5 mmole) avec l'orthoacétate de triéthyle (47,2 ml = 42,1 g, 260 mmole, 8 éq.) a été chauffé à 145° (temp. bain d'huile 160°) sous courant d'azote, dans un réacteur équipé d'une colonne Vigreux et d'un condensateur, pendant que l'on ajoutait continuellement, durant 4 h, de l'acide pivalique (232 mg, 2,28 mmole, 7 mole %) dans l'orthoacétate de triéthyle (5,9 ml = 5,26 g, 32,5 mmole, 1 éq.). Pendant la réaction, l'éthanol libéré, ainsi que l'acétate d'éthyle et un peu d'orthoacétate, a été enlevé par distillation. Le mélange de la réaction a été chauffé pendant encore 1 h, ensuite refroidi à 20°, hydrolysé à l'aide de $NaHCO_3$ aqueux dilué et extrait à l'éther. La phase organique a été lavée deux fois à l'eau et une fois avec saumure, séchée sur $Na_2SO_4$ et évaporée pour fournir 15,4 g de produit brut. Après distillation des têtes (6,6 g, 50°/1,1x$10^2$ Pa), on a obtenu 5,30 g (95°/1,1x$10^2$ Pa) de (+)-(1R)-2-pentyl-2- cyclopentène-1-acétate d'éthyle (rend. 73%) ayant une pureté énantiomérique de 90% e.e. (colonne chirale de type Megadex® 5).

$$[\alpha]^{20}_D = +23{,}6 \; (c = 3{,}9 \; ; \; CHCl_3)$$

RMN($^1$H, 360MHz): 0,89(t, J=7, 3H); 1,26(t, J=7, 3H); 1,22-1,63(m, 7H); 1,86-2,36(m, 6H); 2,53(dd, J=15 et 4, 1H); 2,95(large, 1H); 4,14(q, J=7, 2H); 5,37(large, s, 1H) δppm.
RMN($^{13}$C): 14,1(q); 14,3(q); 22,6(t); 27,4(t); 28,9(t); 30,4(t); 30,5(t); 31,8(t); 38,8(t); 43,7(d) 60,2(t); 124,3(d); 146,3 (s); 173,4(s) δppm.
SM : 224($M^+$, 24), 176(5), 150(31), 136(68), 121(20), 107(30), 93(56), 80(100), 79(92), 67(58), 55(18), 41(46), 29 (63).

Exemple 3

Préparation du (+)-(1R)-2-pentyl-2-cyclopentène-1-acétate de méthyle

On a transformé le cyclopenténol décrit auparavant de façon similaire à celle décrite dans l'Exemple 2, mais en utilisant l'orthoacétate de triméthyle (27 éq.) et 10% molaire d'acide pyvalique. La réaction a été réalisée à une température d'environ 115°, avec une durée d'environ 6 h. Après le traitement usuel, on a obtenu le (+)-(1R)-2-peutyl-2-cyclopentène-1-acétate de méthyle désiré avec 43% de rendement et une pureté énantiomérique de 90% e.e. (colonne chirale Megadex® 5).
Il présentait les données analytiques suivantes

$$[\alpha]^{20}_D = +25{,}3 \; (c = 3{,}6 \; ; \; CHCl_3)$$

RMN($^1$H, 360MHz) : 0,89(t, J=7, 3H) ; 1,20-1,60(m, 7H) ; 1,85-2,35(m, 6H) ; 2,54(dd, J=15 et 4, 1H) ; 2,93(large, 1H) ; 3,67(s, 3H) ; 5,37(large s, 1H) δppm.
RMN($^{13}$C): 14,1(q) ; 22,6(t) ; 27,4(t); 28,9(t) ; 30,4(t) ; 30,5(t) ; 31,8(t); 38,5(t) ; 43,6(d) ; 51,5(q) ; 124,4(d) ; 146,2 (s) ; 173,8(s) δppm.
SM: 210($M^+$, 32), 178(3), 150(16), 136(59), 121(13), 107(23), 93(43), 80(100), 67(42), 41(36), 29(31).

Cet ester a également été préparé, avec une pureté chimique et optique identique, par trans-estérification de son homologue décrit à l'Exemple 2 (rend. 85%), ou saponification du même homologue pour former l'acide correspondant, qui a ensuite été estérifié (rend. global 85% ; conditions conventionnelles).

L'acide (+)-(1R)-2-pentyl 2-cyclopentène-1-acétique intermédiaire (rend. 89%) présentait une pureté énantiomérique de 90% e.e. et les données analytiques suivantes :

$$[\alpha]^{20}_{D} = +32{,}5 \ (c = 6{,}0 \ ; \ CHCl_3)$$

RMN($^1$H, 360MHz) : 0,89(t, J=7, 3H); 1,20-1,65(m, 7H); 1,86-2,36(m, 6H); 2,60(dd, J=15,5 et 4, 1H); 2,95(large, 1H); 5,39(large s, 1H); 10,3-11,3(large, 1H) δppm.

RMN($^{13}$C): 14,1(q); 22,6(t); 27,4(t); 28,9(t); 30,4(t); 30,5(t); 31,8(t); 38,5(t); 43,4(d); 124,6(d); 146,0(s); 180,2(s) δppm.

SM : 196($M^+$, 31), 136(56), 125(10), 121(12), 107(20), 93(49), 91(41), 79(100), 77(62), 67(40), 53(21), 41(48), 29 (39).

Exemple 4

Préparation du (+)-(1R,2S,3R)-2,3-époxy-2-pentyl-1-cyclopentaneacétate de méthyle

Une solution de l'ester décrit à l'Exemple 3 (2,10 g, 10,0 mmole) et d'anhydride maléique (1,46 g, 15,0 mmole, 1,5 éq.) dans $CH_2Cl_2$ (15 ml) a été traitée goutte-à-goutte à 10° avec $H_2O_2$ à 70% (0,58 g, 12 mmole, 1,2 éq.). Après 4 h à 10°, le mélange de la réaction a été versé sur une solution aqueuse saturée de $NaHCO_3$. Le produit a été extrait à l'éther, lavé à l'eau et saumure, séché sur $Na_2SO_4$ et évaporé pour fournir 2,35 g de produit brut. Distillation au four à boules (temp. four 70°/8 Pa) a fourni l'époxyde en titre à l'état pur (1,95 g, 86%), avec un excès diastéréomérique e.d. de 100% et une pureté énantiomérique de 90% e.e. (colonne Megadex® 5).

Un résultat identique a été obtenu lorsqu'on a utilisé 70% $H_2O_2$ (360 mg, 7,5 mmole), ajouté à une solution de $(CF_3CO)_2O$ (2,31 g = 1,53 ml, 11,0 mmole) dans $CH_2Cl_2$ (20 ml) à 0°. Après 30 min, cette solution a été ajoutée goutte-à-goutte, à -50°, à une suspension de l'ester décrit dans l'Exemple 3 (1,05 g, 5,0 mmole) et de $Na_2CO_3$ (1,59 g, 15 mmole) dans le $CH_2Cl_2$ (10 ml). Une fois l'addition terminée (15 min), la réaction était complète. La solution a été traitée comme décrit auparavant et le produit distillé pour fournir 863 mg (rend. 76%) de (+)-(1R,2S,3R)-2,3-époxy-2-pentyl-1-cyclopentaneacétate de méthyle pur, ayant des caractéristiques identiques à celles citées plus haut.

$$[\alpha]^{20}_{D} = +13{,}3 \ (c = 3{,}7 \ ; \ CHCl_3)$$

RMN($^1$H, 360MHz) : 0,89(t, J=7, 3H); 1,11(m, 1H); 1,22-1,40(m, 6H); 1,48-1,66(m, 2H); 1,76-1,91(m, 2H); 1,95 (dd, J=14 et 8, 1H); 2,22-2,38(m, 2H); 2,60(dd, 15,5 et 4, 1H); 3,29(s, 1H); 3,66(s, 3H) δppm.

RMN($^{13}$C): 14,0(q); 22,6(t); 24,7(t); 26,5(t); 26,7(t); 29,2(t); 31,9(t); 34,5(t); 37,6(d); 51,6(q); 62,6(d); 68,3(s); 173,6 (s) δppm.

SM : 226($M^+$, 3), 211(12), 183(26), 167(35), 152(42), 138(22), 123(37), 109(51), 96(89), 81(100), 67(56), 55(72), 41(88), 29(49).

Par ailleurs, d'autres conditions de réaction ont encore été utilisées. Le tableau ci-après résume et compare les résultats décrits ci-dessus avec ceux obtenus en variant les conditions d'époxydation. Les différents composés (configurations absolues) présents dans le produit de la réaction (% en poids), ainsi que leur temps de rétention sur colonne de type DB-WAX [100° (3 min), puis 30°/min] sont indiquées au tableau.

## TABLEAU I

| Essai | Conditions de réaction | Produits (%) | | | | |
|---|---|---|---|---|---|---|
| | | 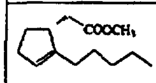 | 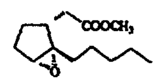 | 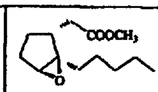 | 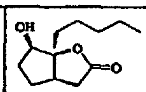 | 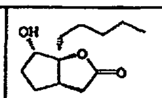 |
| | | (5'37) | (6'59) | (6'68) | (10'24) | (13'02) |
| 1 | $(CF_3CO)_2O/H_2O_2/Na_2CO_3/CH_2Cl_2/-50°$ 10 min | - | 97 | - | 1 | - |
| | extr. | | 91 | | 2 | 1 |
| 2 | anhydride maléïque/ $H_2O_2/CH_2Cl_2/10°$ 2 h | 32 | 58 | 1 | - | 6 |
| | 4 h ; extr. | - | 84 | - | 1 | 10 |
| 3 | anhydride maléïque/ $H_2O_2$/toluène/10→30° 1 h | 3 | 77 | 6 | - | 5 |
| | 4 h extr. | 1 | 80 | 1 | - | 9 |
| 4 | HCOOH/HCOONa/ $H_2O_2/40°$ 6 h | 72 | 21 | 4 | pas dét. | |
| 5 | acide m-chloroperbenzoïque/$CH_2Cl_2/0°$ 15 min extr. | - | 83 | 14 | - | - |
| 6 | $CH_3CO_3H/CH_2Cl_2/20°$ 1 h | 4 | 80 | 9 | - | - |
| | 2 h | 1 | 78 | 12 | - | - |
| | extr. | - | 83 | 12 | - | - |

Exemple 5

Préparation du (+)-(1R,2S,3R)-2-époxy-2-pentyl-1-cyclopentaneacétate d'éthyle

Cet époxyde a été préparé dans des conditions similaires à celles décrites dans l'Exemple 4, mais en partant de l'ester décrit à l'Exemple 2. Le tableau ci-après résume les résultats obtenus dans les différentes conditions de réaction.

## TABLEAU II

| Essai | Conditions de réaction | Produits (%) | | | | |
|---|---|---|---|---|---|---|
| | | 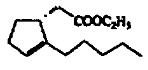 | 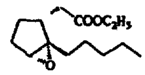 | 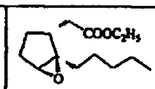 | 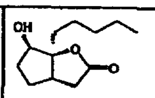 | 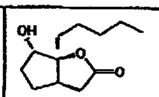 |
| 1 | $(CF_3CO)_2O/H_2O_2/$ $Na_2CO_3/CH_2Cl_2/-50°$ 10 min | - | 91 | - | - | - |
| 2 | anhydride maléïque/ $H_2O_2/CH_2Cl_2/10°$ 2 h | - | 82 | - | 1 | 11 |
| 3 | acide m-chloroper-benzoïque/$CH_2Cl_2/0°$ 15 min extr. | - | 77 | 13 | - | - |
| 4 | $CH_3CO_3H/CH_2Cl_2/20°$ 1 h | 3 | 83 | 12 | - | - |
| | 2 h | 1 | 84 | - | - | 11 |

Exemple 6

Préparation de (+)-(1R)-cis-3-oxo-2-pentyl-1-cyclopentaneacétate de méthyle ou (±)-(1R)-cis-Hédione®

Un mélange de l'époxyde décrit à l'Exemple 4 (1,0 g, 4,42 mmole), tamis moléculaires de 4 Å (0,50 g) et toluène (10 ml) a été traité à 23° avec de l'éthérate de trifluorobore $BE_3.OEt_2$ (56 µl = 63 mg, 0,44 mmole, 0,1 éq.). Après 8 min à 20-25°, la réaction a été arrêtée en ajoutant une solution aqueuse saturée de bicarbonate de sodium glacée et le produit extrait à l'éther, lavé deux fois à l'eau et une fois à la saumure, séché sur $Na_2SO_4$ et évaporé pour fournir 1,09 g de produit brut. Distillation de ce dernier au four à boules (temp. four 60-85°/7x$10^2$ Pa) a fourni 824 g de (+)-(1R)-cis-Hédione® à 80% pure (rend. 66%, 87% e.d., 90% e.e.). Une deuxième distillation fractionnée a fourni la (+)-(1R)-cis-Hédione® contenant 5% en poids d'isomère de configuration (-)-(1S)-trans, ainsi que 5% en poids d'un diène secondaire à savoir le (2-pentyl-2-cyclopentén-1-ylidène)acétate de méthyle.

Lorsqu'on a utilisé, selon la même méthode, des quantités stoïchiométriques de $BF_3.OEt_2$, on a obtenu la (+)-(1R)-cis-Hédione® pure à 90% (90% d.e., 90% e.e.), avec 70% de rendement.

Par ailleurs, on a procédé de façon analogue mais en utilisant des conditions de réaction différentes de celles mentionnées ci-dessus. Le tableau ci-après résume et compare les résultats susmentionnés avec ceux obtenus avec d'autres conditions de réaction. Les différents composés (configurations absolues) présents dans le produit de la réaction (% en poids), ainsi que leur temps de rétention sur colonne de type DB-WAX (100° (3 min), puis 30°/min) sont indiqués au tableau.

## TABLEAU III

| Essai | Conditions de réaction | Composés (% en poids) | | | | |
|---|---|---|---|---|---|---|
| | | 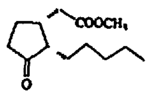 | 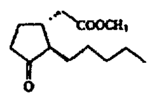 | 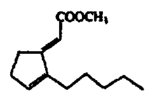 | 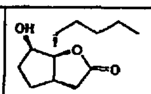 | Epoxyde de départ |
| | | (7'48) | (7'30) | (6'70) | (10'24) | |
| 1 | $AlCl_3$ (0,1 éq.), $CH_2Cl_2$,5° 10 min | 86 | 6 - 7 | 5 | 2 | - |
| 2 et 3 | $BF_3.OEt_2$ (1 éq.), tol.,20° 10 min | 88 | 4 | 8 | - | - |
| | a) tamis moléculaires 4 Å (50%) b) $BF_3.OEt_2$ (0,1 éq.), tol., 20° 5 min | 82 | 6 | 9 | 2 | - |
| 4 | $MgI_2$ (1 éq.), tol.,110° 10 min | 60 | 2 | 16 | - | - |
| 5 | Filtrol® G24 (10%), tol., 110° 30 min | 56 | 5 | 9 | 20 | - |
| 6 | $Sc(OSO_2CF_3)_3$ (0,5 éq.), $CH_2Cl_2$, 20° 15 min | 78 | 6 | 7 | 9 | 0 |
| 7 | $MgBr_2$, tol., 110° 3 h. | 1 | - | - | 1 | 88 |
| 8 | $LiClO_4$, $Et_2O$, 5° 24 h | 24 | - | 23 | 23 | 28 |
| 9 | $LiClO_4$, tol., 24° 63 h | 54 | 1 | 23 | 5 | 5 |
| 10 | $MgClO_4$, tol., 50° 1 h | 31 | - | 34 | - | 35 |

Exemple 7

Préparation de (S)-tétrahydro-1-méthyl-3-phényl-1H,3H-pyrrolo[1,2-c][1,3,2] oxazaborole

Une solution de (1R,2'S)-1-phényl-2'-pyrrolidineméthanol (obtenu selon Ookawa et al., ref. citée) (0,554 g, 3,1 mmole) et de triméthylboroxine (0,226 g, 2,1 mmole) dans le toluène a été agitée à 25°C. Après 45 min, la solution a été lentement chauffée à reflux pendant 2 h, et l'eau et l'acide boronique formé ont été enlevés par distillation azéotropique avec du toluène. Après concentration du mélange à moitié, du toluène frais (ca. 5 ml) a été rajouté (il était nécessaire d'ajouter 3 ou 4 volumes de toluène pendant la réaction). On a laissé refroidir le mélange réactionnel à température ambiante pendant la nuit, et la solution de l'oxazaborolidine souhaité (5,2 ml, ca. 0,59 M) a été transférée dans une bouteille, en utilisant une seringue, et stockée sous argon. La solution pouvait être utilisée telle quelle dans les réactions de réduction décrite auparavant.

SM : 201($M^+$, 100), 200(94), 186(12), 172(92), 158(20), 130(42), 104(19), 91(78), 77(18), 70(18), 67(18), 51(13),

39(15).

Exemple comparatif

Procédé décrit dans WO 95/33735

**A.** On a procédé à l'époxydation du 2-pentyl-2-cyclopentène-1-acétate de méthyle dans les conditions décrites dans le document cité ci-dessus, Exemple 1 ($Na_2SO_4$, HCOOH, HCOONa, $H_2O_2$ à 70%, 40°, 6 h). Après extraction à l'éther et distillation au four à boules du produit de la réaction, on a obtenu essentiellement l'ester de départ, comme il ressort du tableau I présenté plus haut (voir essai 4), le rendement en l'époxyde désiré étant seulement de ~20%.

**B.** On a transformé le c-2,3-époxy-2-pentyl-r-1-cyclopentaneacétate de méthyle (obtenu de façon analogue à celle décrite à l'Exemple 4 mais en partant du 2-pentyl-2-cyclopentène-1-acétate de méthyle racémique) de façon à analogue à celle décrite à l'exemple 2 de WO 95/33735. Un mélange de 400 mg de l'époxyde ci-dessus (1,80 mmole) et de 19 mg de LiI (0,14 mmole) a été placé dans un four à boules préchauffé à 190°. Après 10 min, le mélange noir a été distillé sous vide (8 Pa). Le distillat (277 mg) consistait essentiellement en l'époxyde de départ (82% pur). On l'a combiné à nouveau avec le résidu (68 mg) et chauffé 30 min à 190°, refroidi et distillé au four à boules (temp. four 90°/8 Pa) pour obtenir 110 mg de distillat. Redistillation de ce produit (temp. four 110°/11 Pa) a fourni 85 mg d'un produit pur à 90% présentant les données analytiques suivantes :

IR (pur) : 2950, 2920, 2855, 1760, ~1735(épaulement), ~1705(épaulement), 1450, 1215, 1185 $cm^{-1}$.
RMN($^1H$, 360MHz): 0,87(t, J=7, 3H); ~1,2-1,8(m, 12H) ; 1,90(m, 1H); 2,05(m, 1H); 2,28(dd, J=17,5 et 3,5, 1H); 2,51(m, 1H); 2,85(dd, J=17,5 et 10, 1H) δppm.
RMN($^{13}C$): 177,5(s) ; 98,3(s); 42,1(d); 39,4(t); 38,1(t); 37,1(t); 34,4(t); 32,1(t); 24,0(t); 23,9(t); 22,5(t) ; 14,0(q) δppm.
SM: 196($M^+$, 6), 167(6), 153(10), 140(17), 125(100), 97(70), 81(18), 71(26), 69(30), 55(50), 41(69).

Ces données spectrales correspondent à la lactone dont la structure est citée plus haut dans le texte.

## Revendications

1. Epoxyde de formule

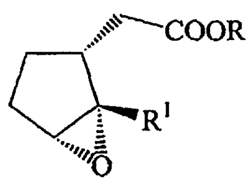

(I)

ayant une configuration cyclanique (1R), le groupe en position 2 étant en configuration trans et le groupe époxy étant en configuration cis par rapport à celui en position 1 du cycle, et dans laquelle R représente un radical alkyle linéaire ou ramifié de $C_1$ à $C_4$ et $R^1$ représente un radical d'hydrocarbure, saturé ou insaturé, linéaire ou ramifié, ayant de 1 à 8 atomes de carbone.

2. (+)-(1R,2S,3R)-2,3-Epoxy-2-pentyl-1-cyclopentaneacétate de méthyle.

3. Utilisation d'un époxyde selon la revendication 1, pour la préparation d'une cétone cyclique de formule

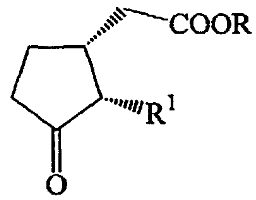

(II)

ayant une configuration cyclanique (1R)-cis et dans laquelle les symboles R et $R^1$ ont le sens indiqué à la formule (I), **caractérisée en ce qu'**on traite ledit époxyde, dans un solvant organique inerte, avec un agent acide constitué par un acide de Lewis approprié ou une terre argileuse acide.

**4.** Utilisation selon la revendication 3, du (+)-(1R,2S,3R)-2,3-époxy-2-pentyl-1-cyclopentaneacétate de méthyle, pour préparer le (+)-(1R)-cis-3-oxo-2-pentyl-1-cyclopentaneacétate de méthyle.

**5.** Utilisation selon la revendication 3 ou 4, **caractérisée en ce que** l'agent acide est l'éthérate de trifluorobore ou le trichlorure d'aluminium.

**6.** Procédé pour la préparation d'un époxyde selon la revendication 1, **caractérisé en ce qu'**on fait réagir un ester de formule

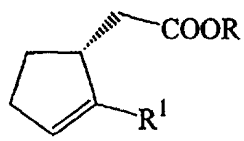

(III)

ayant une configuration cyclanique (1R) et dans laquelle les symboles R et $R^1$ ont le sens indiqué à la formule (I), avec un péracide fort, dans un solvant organique inerte.

**7.** Procédé selon la revendication 6, **caractérisé en ce que** l'ester de formule (III) est obtenu par une méthode qui comprend la réduction d'une cétone de formule

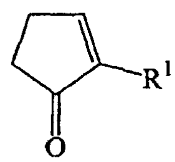

(V)

dans laquelle $R^1$ a le sens indiqué à la formule (III), à l'aide de $BH_3$, en présence d'une oxazaborolidine chirale, pour obtenir un cyclopenténol de configuration (1R) de formule

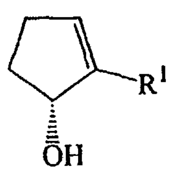

(IV)

que l'on fait ensuite réagir avec un orthoacétate d'alkyle approprié, dans des conditions de réaction propices à un réarrangement de type Claisen, pour fournir l'ester désiré.

**8.** Procédé selon la revendication 7, **caractérisé en ce que** la cétone est réduite en utilisant une oxazaborolidine de

formule

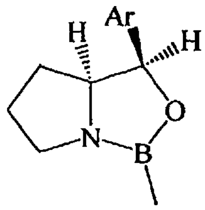

(VI)

dans laquelle Ar représente un groupe phényle ce phényle substitué sélectionné dans le groupe constitué par le toluène, le xylène, le di-tert-butylbenzène, le mésitylène, ou groupe aryle condensé.

**9.** Procédé selon la revendication 6, **caractérisé en ce qu'**on fait réagir le (+)-(1R)-2-pentyl-2-cyclopentène-1-acétate de méthyle pour obtenir le (+)-(1R,2S,3R)-2,3-époxy-2-pentyl-1-cyclopentaneacétate de méthyle.

**10.** Procédé selon la revendication 6 ou 9, **caractérisé en ce que** le péracide fort est l'acide permaléique ou l'acide trifluoroperacétique.

**11.** Procédé selon la revendication 7 ou 8, **caractérisé en ce qu'**on réduit la 2-pentyl-2-cyclopentén-1-one pour former le (+)-(1R)-2-pentyl-2-cyclopentén-1-ol.

**12.** Procédé selon la revendication 11, **caractérisé en ce qu'**on fait réagir ledit cyclopenténol avec l'orthoacétate de méthyle, en présence d'acide pyvalique, pour obtenir le (+)-(1R)-2-pentyl-2-cyclopentène-1-acétate de méthyle.

**13.** Ester de formule

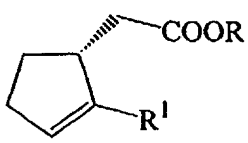

(III)

ayant une configuration cyclanique (1R) et dans laquelle le symbole R représente un radical alkyle linéaire ou ramifié de $C_1$ à $C_4$ et le symbole $R^1$ représente un radical d'hydrocarbure, saturé ou insaturé, linéaire ou ramifié, ayant de 1 à 8 atomes de carbone.

**14.** (+)-(1R)-2-Pentyl-2-cyclopentène-1-acétate de méthyle.

**15.** Epoxyde de formule

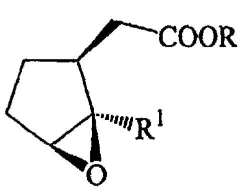

(I')

dans laquelle les groupes substituants époxy et carboxy ont une configuration relative strictement cis, R représente un radical alkyle linéaire ou ramifié de $C_1$ à $C_4$ et $R^1$ représente un radical d'hydrocarbure, saturé ou insaturé, linéaire ou ramifié, ayant de 1 à 8 atomes de carbone.

## Patentansprüche

1. Epoxid der Formel

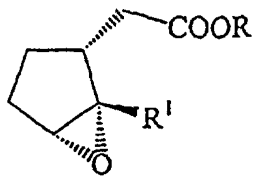

(I)

mit einer (1R)-Cyclankonfiguration, wobei die Gruppe in Position 2 in einer trans-Konfiguration und die Epoxidgruppe in einer cis-Konfiguration bezüglich derjenigen in der Position 1 des Rings vorliegt, und in der R für einen geradkettigen oder verzweigten $C_1$-$C_4$-Alkylrest steht und $R^1$ für einen gesättigten oder ungesättigten, geradkettigen oder verzweigten Kohlenwasserstoffrest mit von 1 bis 8 Kohlenstoffatomen steht.

2. (+)-(1R,2S,3R)-2,3-Epoxy-2-pentyl-1-cyclcopentanessigsäuremethylester.

3. Verwendung eines Epoxids nach Anspruch 1 zur Herstellung eines cyclischen Ketons der Formel

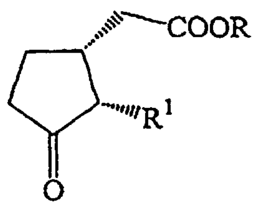

(II)

mit einer (1R)-cis-Cyclankonfiguration, in der die Symbole R und $R^1$ die in Formel (I) angegebene Bedeutung haben, **dadurch gekennzeichnet, daß** das Epoxid in einem inerten organischen Lösungsmittel mit einem sauren Mittel behandelt wird, das aus einer geeigneten Lewis-Säure oder einer sauren Tonerde besteht.

4. Verwendung nach Anspruch 3 von (+)-(1R,2S,3R)-2,3-Epoxy-2-pentyl-1-cyclopentanessigsäuremethylester für die Herstellung von (+)-(1R)-cis-3-Oxo-2-pentyl-1cyclopentanessigsäuremethylester.

5. Verwendung nach Anspruch 3 oder 4, **dadurch gekennzeichnet, daß** das saure Mittel Trifluorboretherat oder Aluminiumtrichlorid ist.

6. Verfahren zur Herstellung eines Epoxids nach Anspruch 1, **dadurch gekennzeichnet, daß** ein Ester der Formel

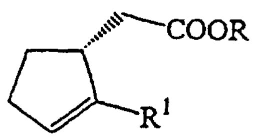

(III)

mit einer (1R)-Cyclankonfiguration, in der die Symbole R und $R^1$ die in Formel (I) angegebene Bedeutung haben, mit einer starken Persäure in einem inerten organischen Lösungsmittel umgesetzt wird.

7. Verfahren nach Anspruch 6, **dadurch gekennzeichnet, daß** der Ester der Formel (III) mittels eines Verfahrens hergestellt wird, das die Reduktion eines Ketons der Formel

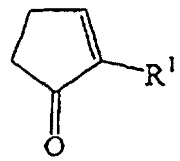

(V)

umfaßt, in der $R^1$ die in Formel (III) angegebene Bedeutung hat, mit Hilfe von $BH_3$ in Gegenwart eines chiralen Oxazaborolidins, um ein Cyclopentenol mit der Konfiguration (1R) der Formel

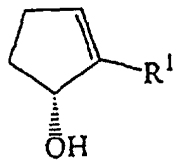

(IV)

zu erhalten, das anschließend mit einem geeigneten Orthoessigsäurealkylester unter Reaktionsbedingungen umgesetzt' wird, die für eine Claisen-Umlagerung günstig sind, um den gewünschten Ester zu liefern.

**8.** Verfahren nach Anspruch 7, **dadurch gekennzeichnet, daß** das Keton unter Verwendung eines Oxazaborolidins der Formel

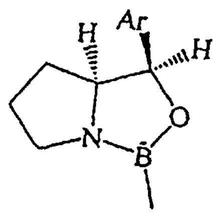

(VI)

umgesetzt wird, in der Ar für eine Phenylgruppe oder substituierte Phenylgruppe, die in der aus Toluol, Xylol, Di-tert-butylbenzol, Mesitylen bestehenden Gruppe ausgewählt ist, oder eine kondensierte Arylgruppe steht.

**9.** Verfahren nach Anspruch 6, **dadurch gekennzeichnet, daß** (+)-(1R)-2-Pentyl-2-cyclopenten-1-essigsäuremethylester umgesetzt wird, um (+)-(1R,2S,3R)-2,3-Epoxy-2-pentyl-1-cyclopentanessigsäuremethylester zu erhalten.

**10.** Verfahren nach Anspruch 6 oder 9, **dadurch gekennzeichnet, daß** die starke Persäure Permaleinsäure oder Trifluorperessigsäure ist.

**11.** Verfahren nach Anspruch 7 oder 8, **dadurch gekennzeichnet, daß** 2-Pentyl-2-cyclopenten-1-on reduziert wird, um (+)-(1R)-2-Pentyl-2-cyclopenten-1-ol zu bilden.

**12.** Verfahren nach Anspruch 11, **dadurch gekennzeichnet, daß** das Cyclopentenol mit Orthoessigsäuremethylester in Gegenwart von Pivalinsäure umgesetzt wird, um (+)-(1R)-2-Pentyl-2-cyclopenten-1-essigsäuremethylester zu erhalten.

**13.** Ester der Formel

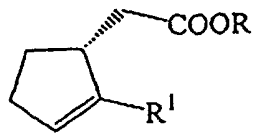

(III)

mit einer (1R)-Cyclankonfiguration, in der das Symbol R für einen geradkettigen oder verzweigten $C_1$-$C_4$-Alkylrest steht und das Symbol $R^1$ für einen gesättigten oder ungesättigten, geradkettigen oder verzweigten Kohlenwasserstoffrest mit von 1 bis 8 Kohlenstoffatomen steht.

**14.** (+)-(1R)-2-Pentyl-2-cyclopenten-1-essigsäuremethylester.

**15.** Epoxid der Formel

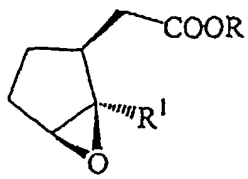

(I')

in der die Epoxy- und Carboxy-Substituentengruppen eine strenge cis-Relativkonfiguration aufweisen, R für einen geradkettigen oder verzweigten $C_1$-$C_4$-Alkylrest steht, und $R^1$ für einen gesättigten oder ungesättigten, geradkettigen oder verzweigten Kohlenwasserstoffrest mit von 1 bis 8 Kohlenstoffatomen steht.

## Claims

**1.** Epoxide of formula

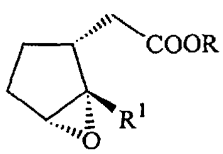

(I)

having a cyclanic (1R) configuration, the group in position 2 being in a trans configuration and the epoxy group being in a cis configuration with respect to that in position 1 of the ring, and in which R represents a $C_1$ to $C_4$ linear or branched alkyl radical and $R^1$ represents a saturated or unsaturated, linear or branched, hydrocarbon radical having from 1 to 8 carbon atoms.

**2.** (+)-Methyl (1R,2S,3R)-2,3-epoxy-2-pentyl-1-cyclopentaneacetate.

**3.** Use of an epoxide according to claim 1, for the preparation of a cyclic ketone of formula

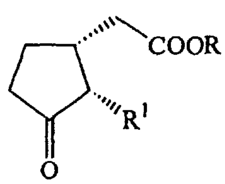

(II)

having a cyclanic configuration (1R)-cis and in which the symbols R and $R^1$ have the meaning indicated in formula

(I), **characterized in that** said epoxide is treated, in an inert organic solvent, with an acidic agent consisting of an appropriate Lewis acid or an acidic clay.

**4.** Use according to claim 3, of (+)-methyl (1R,2S,3R)-2,3-epoxy-2-pentyl-1-cyclopentaneacetate to prepare (+)-methyl (1R)-cis-3-oxo-2-pentyl-1-cyclopentaneacetate.

**5.** Use according to claim 3 or 4, **characterized in that** the acidic agent is trifluoroboron etherate or aluminium trichloride.

**6.** Process for the preparation of an epoxide according to claim 1, **characterized in that** an ester of formula

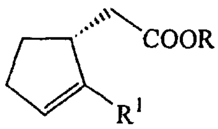

(III)

having a cyclanic configuration (1R) and in which the symbols R and $R^1$ have the meaning indicated in formula (I), is reacted with a strong peracid, in an inert organic solvent.

**7.** Process according to claim 6, **characterized in that** the ester of formula (III) is obtained by a method which comprises the reduction of a ketone of formula

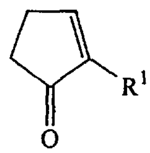

(V)

in which $R^1$ has the meaning indicated in formula (III), by means of $BH_3$, in the presence of a chiral oxazaborolidine, to provide a (1R) configuration cyclopentenol of formula

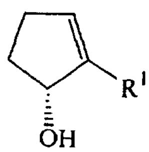

(IV)

which is then reacted with an appropriate alkyl orthoacetate, under reaction conditions favorable for a Claisen type rearrangement, to give the desired ester.

**8.** Process according to claim 7, **characterized in that** the ketone is reduced using a chiral oxazaborolidine according to formula

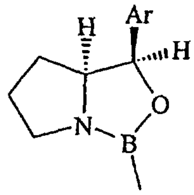

(VI)

in which Ar represents a phenyl group or a substituted phenyl group selected from the group consisting of toluene, xylene, di-tert-butylbenzene, mesitylene or a fused aryl group.

**9.** Process according to claim 6, **characterized in that** is reacted (+)-methyl (1R)-2-pentyl-2-cyclopentene-1-acetate to obtain (+)-methyl (1R,2S,3R)-2,3-epoxy-2-pentyl-1-cyclopentaneacetate.

**10.** Process according to claim 6 or 9, **characterized in that** the strong peracid is permaleic acid or trifluoroperacetic acid.

**11.** Process according to claim 7 or 8, **characterized in that** 2-pentyl-2-cyclopenten- 1-one is reduced to give (+) -(1R)-2-pentyl-2-cyclopenten-1-ol.

**12.** Process according to claim 11, **characterized in that** said cyclopentenol is reacted with methyl orthoacetate, in the presence of pyvalic acid, to obtain (+)-methyl (1R)-2-pentyl-2-cyclopentene-1-acetate.

**13.** Ester of formula

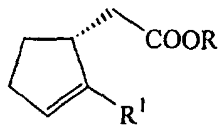

(III)

having a cyclanic (1R) configuration and wherein symbol R represents a $C_1$ to $C_4$ linear or branched alkyl radical and the symbol $R^1$ represents a saturated or unsaturated, linear or branched, hydrocarbon radical having from 1 to 8 carbon atoms.

**14.** (+)-Methyl (1R)-2-pentyl-2-cyclopentene-1-acetate.

**15.** Epoxide of formula

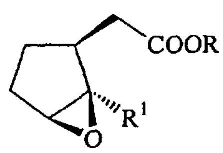

(I')

wherein the substituent groups epoxy and carboxy have a strictly cis configuration, R represents a $C_1$ to $C_4$ linear or branched alkyl radical and $R^1$ represents a saturated or unsaturated, linear or branched, hydrocarbon radical having from 1 to 8 carbon atoms.